# EUROPÄISCHE PATENTSCHRIFT

(11) **EP 0 473 892 B1**
(45) Veröffentlichungstag und Bekanntmachung des Hinweises auf die Patenterteilung: **18.05.1994**
(21) Anmeldenummer: 91110267.1
(22) Anmeldetag: 21.06.1991
(51) Int. Cl.: B05B 11/00, A61L 2/16, A61J 1/00, B65D 81/24

(54) **Fluid-Abgabeeinrichtung für keimfreies Fluid**
Dispenser for aseptic fluid
Distributeur pour fluide aseptique

(30) Priorität: 29.08.1990 DE 4027320
(43) Veröffentlichungstag der Anmeldung: 11.03.1992
(73) Patentinhaber: URSAPHARM ARZNEIMITTEL GMBH, D-66129 Saarbrücken (DE)
(72) Erfinder: Geimer, Günter, W-6797 Schönenberg-Kübelberg (DE)
(74) Vertreter: Körber, Wolfhart, Dr.rer.nat.

(56) Entgegenhaltungen:
- DE-A- 2 830 977
- GB-A- 2 097 376
- PATENT ABSTRACTS OF JAPAN vol. 13, no. 529 (C-658)(3877) 27. November 1989; & JP-A-1 215 364 (SERUTETSUKU LAB K.K.) 29. August 1989

## Beschreibung

Die Erfindung betrifft eine Fluid-Abgabeeinrichtung für keimfreies Fluid nach dem Oberbegriff des Anspruchs 1, wie sie aus der Druckschrift DE-A-2 830 977 bekannt ist.

In der pharmazeutischen Zeitung, 124. Jahrgang, Nr. 20, vom 17. Mai 1979, ist auf Seiten 949 und 950 ebenfalls eine Fluid-Abgabeeinrichtung beschrieben, die die Form einer Tropfpipette aufweist und an einem Augentropfen enthaltenden Behälter befestigt ist. In der Tropfpipette ist ein aus einer Silberschicht oder einem schwerlöslichen Silbersalz bestehendes Silberdepot so eingebracht, daß die mit dem in den Behälter zurücklaufenden Tropfen eingezogenen Anflugkeime einer antimikrobiell (gleich oligodynamisch) wirksame Silberschicht passieren müssen, bevor sie in den Behälter gelangen. Weiterhin ist ausgeführt, daß sich Keramikringe mit eingelagertem Silberchlorid und einem Durchmesser von 9 mm als geeignet erwiesen haben. Diese Keramikringe lassen sich durch einfaches Einschieben in die Tropfer aller apothekenüblichen Augentropfergläser fest installieren. Diese Art der Einbringung des Silberdepots in die Tropfer ist insofern nachteilig, als lediglich die an der Tropferwandung zurücklaufenden Tropfen mit dem Silberdepot in Berührung kommen, nicht jedoch die Flüssigkeitsteilchen im Inneren der nach der üblichen Benutzung des Augentropfenbehälters mit nach unten gerichtetem Tropfer von diesem in den Behälter zurückströmenden Flüssigkeitssäule. Jede Benutzung des Augentropfenbehälters führt somit zu einer Kontaminierung der Augentropfen. Ein weiterer Nachteil besteht darin, daß das Behälterinnere über den Tropfer mit der Außenluft in Verbindung steht, so daß auch während der Nichtbenutzung Keime ständig eindringen und zur Kontamination der Augentropfen im Behälter führen.

Es ist Aufgabe der Erfindung, eine Fluid-Abgabeeinrichtung der eingangs genannten Art so weiterzubilden, daß eine Kontamination des keimfreien Fluids sicher verhindert wird.

Diese Aufgabe wird durch das kombinatiorische Zusammenwirken der kennzeichnenden Merkmale des Anspruchs 1 gelöst.

Der Durchflußquerschnitt des Einlaßventils ist so gering, daß das durchströmende Fluid vollständig mit der oligodynamisch wirksamen Substanz in Berührung kommt, so daß auch bei kurzzeitigem Kontakt sämtliche im Fluid befindlichen Keime abgetötet werden. Eine vergleichbare Wirkung wird erzielt, wenn die oligodynamisch wirksame Substanz im entsprechend engen Zulauf oder Ablauf des Einlaßventils angeordnet ist. Auch diejenigen Keime werden sicher und vollständig abgetötet, die sich in der Restflüssigkeit befinden, die insbesondere bei Einsatz eines als Kolbenventil ausgebildeten Einlaßventils und bei Verwendung höherer Viskoseflüssigkeit im Einlaßventil zurückbleibt und eine Verbindung zwischen der Außenluft und dem Fluidvorrat im Vorratsbehälter herstellt. Hier ergibt sich eine besonders intensive Keimabtötung infolge des länger dauernden Kontakts der Flüssigkeit mit der oligodynamisch wirksamen Substanz. Die Dosierpumpe arbeitet luftausgleichsfrei und verhindert somit eine Kontaminierung des Fluidvorrats über die bei der Betätigung herkömmlicher Dosierpumpen in den Vorratsbehälter einströmender und den Druckausgleich herstellenden Luft. Die erfindungsgemäße Fluid-Abgabeeinrichtung sichert die Aufrechterhaltung der Keimfreiheit des im Vorratsbehälter befindlichen Fluids, so daß dieses weder mit Konservierungsstoffen versetzt noch innerhalb des Vorratsbehälters der oligodynamisch wirksamen Substanz ausgesetzt zu werden braucht.

Die besonders intensive Keimabtötung infolge eines länger dauernden Kontakts des Fluids mit der oligodynamisch wirksamen Substanz kann insbesondere bei einem als Sitzventil ausgebildeten Einlaßventil und bei Verwendung niedrigviskoser Flüssigkeit dadurch erzielt werden, daß der Durchgangskanal zumindest im Bereich des Einlaßventils ständig mit dem Fluid gefüllt ist.

Als unterstützende Maßnahme zur Aufrechterhaltung der Keimfreiheit ist die oligodynamisch wirksame Substanz vorzugsweise in einem die Abgabeöffnung verschließbaren Auslaßventil und/oder in dessen Zulauf und/oder Ablauf angeordnet. Das Eindringen von Keimen in dieses Auslaßventil kann durch Abdeckung der Abgabeöffnung mit einer Verschlußkappe erzielt werden, an deren Innenseite die oligodynamisch wirksame Substanz angeordnet ist.

Vorteilhafterweise ist die oligodynamisch wirksame Substanz am Verschlußglied des Einlaßventils und/oder Auslaßventils angeordnet oder sie stellt zumindest einen Teil desselben dar.

Die oligodynamisch wirksame Substanz kann statt dessen oder zusätzlich an dem mit dem Verschlußglied des Einlaßventils und/oder Auslaßventils zusammenwirkenden Ventilsitz/ bzw. Ventilgehäuse angeordnet oder zumindest Teil desselben sein.

Auch eine Anordnung der oligodynamisch wirksamen Substanz an einem Verschlußglied des Einlaßventils und/oder Auslaßventils beaufschlagenden Fehler ist möglich. Ferner kann die oligodynamisch wirksame Substanz auf zumindest einem Teilstück des als Steigrohr ausgebildeten Zulaufs des Einlaßventils angeorndet oder zumindest Teil desselben sein.

Günstig ist auch eine Ausbildung, gemäß welcher die oligodynamisch wirksame Substanz im Bereich zwischen beiden Ventilen auf zumindest einem Teilstück des Durchgangskanals angeordnet oder zumindest Teil desselben ist.

Die oligodynamisch wirksame Substanz kann in einem Trägermaterial, vorzugsweise Korund, eingelagert sein.

Vorzugsweise wird Silber oder eine Legierung desselben in metallischer Form oder in Form einer Verbindung, beispielsweise als Salz, als oligodynamisch wirksame Substanz verwendet.

Silber weist den ppb-Konzentrationen den günstigsten therapeutischen Index auf. Es können aber auch andere Schwermetalle, wie beispielsweise Kadmium, Kupfer oder Messung etc. verwendet werden.

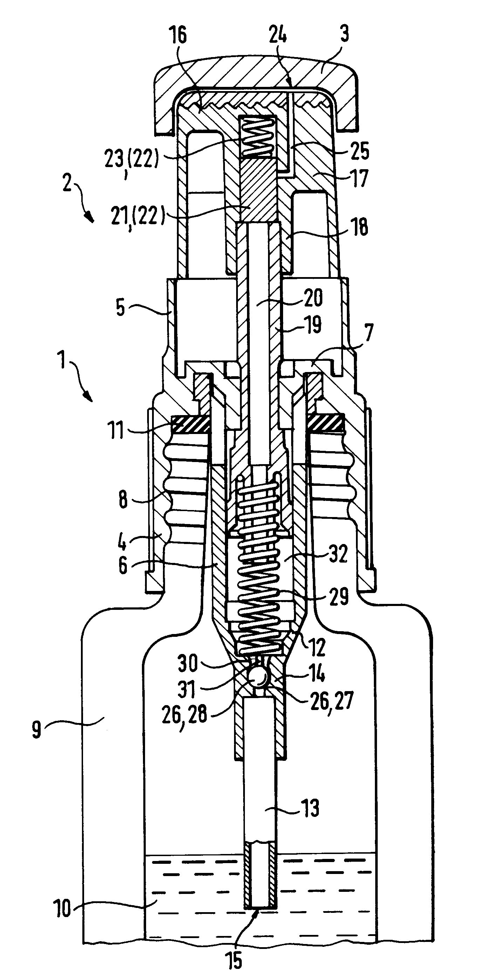

Nachstehend ist die Erfindung anhand eines bevorzugten Ausführungsbeispiels unter Bezugnahme auf die einzige Figur näher beschrieben.

Die Figur zeigt im Längsschnitt eine Dosierpumpe bestehend aus einem zylindrischen Pumpenkörper 1, einem Betätigungskörper 2 und einer Verschlußkappe 3.

Der Pumpenkörper 1 umfaßt einen ersten hohlzylindrischen und in der Zeichnung nach unten offenen Pumpenkörperabschnitt 4, einen Zweiten hohlzylindrischen und in der Zeichnung nach oben offenen Pumpenkörperabschnitt 5 mit reduziertem Durchmesser, sowie einen an beiden Enden offenen Hohlzylinder 6, der zentrisch an einem nach innen gerichteten Ringflansch 7 im Übergangsbereich zwischen beiden Pumpenkörperabschnitten 4,5 befestigt ist. Der erste Pumpenkörperabschnitt 4 weist ein Innengewinde 8 zur Schraubbefestigung eines lediglich angedeuteten Vorratsbehälters 9 auf, der mit einer keimfreien Flüssigkeit, im vorliegenden Fall Augentropfen, gefüllt ist. Zur Sicherung des luftdichten Abschlusses zwischen Vorratsbehälter 9 und Pumpenkörper 4 ist an der in der Zeichnung unteren Seite des Ringflansches 7 eine Dichtungsanordnung 11 vorgesehen. Im Austrittsbereich aus dem ersten Pumpenkörperabsschnitt 4 weist der Hohlzylinder 6 einen sich konisch verjüngenden Übergangsabschnitt 12 auf, an den sich ein zu einem Steigrohr 13 führender zylindrischer Ventilabschnitt 14 reduzierten Durchmessers anschließt. Das freie untere Ende des Steigrohres 13 stellt die Einlaßöffnung 15 der Dosierpumpe dar.

Der Betätigungskopf 2 besteht aus einem in der Zeichnung unten offenen und obenseitig durch ein Kopfstück 16 verschlossenen äußeren hohlen Zylinderabschnitt 17 sowie einem sich zentrisch vom Kopfstück 16 nach unten erstreckenden inneren hohlen Zylinderabschnitt 18. Der Durchmesser des äußeren hohlen Zylinderabschnitts 17 ist geringer als derjenige des ersten Pumpenkörperabschnitts 4.

Ein im Hohlzylinder 6 passend geführter Kolben 19 mit einer Durchgangsbohrung 20 ist mit seinem oberen Endstück im inneren hohlen Zylinderabschnitt 18 befestigt. Ein in letzterem passend geführter Kolbenschieber 21 eines Auslaßventils 22 stützt sich einerseits auf dem Endstück des Kolbens 19 und andererseits über eine Feder 23 am Kopfstück 16 ab. Ein am Kopfstück 16 mit einer Abgabeöffnung 24 ausmindernder Auslaßkanal 25 ist in Höhe des Kolbenschiebers 21 an den Innenraum des inneren hohlen Zylinderabschnitts 18 angeschlossen.

Im Ventilabschnitt 14 ist ein Einlaßventil 26 mit einer mit einem Ventilsitz 27 zusammenwirkenden Kugel 28 ausgebildet. Einer am Kolben 19 befestigte Feder 29 stützt sich an einem Vorsprung 30 am Ventilabschnitt 14 ab und beaufschlagt über einen Stift 31 die Kugel 28. Der Innenraum des Hohlzylinders 6 zwischen dem Kolben 19 und dem Ventilabschnitt 14 ist mit dem Bezugszeichen 32 bezeichnet.

Die Ventilkugel 28 sowie der Kolbenschieber 21 bestehen aus Korund mit einer eingelagerten Substanz mit oligodynamischer Wirkung. In vorliegendem Falle Silber. Zusätzlich kann der Ventilsitz 27 und die Innenseite des inneren hohlen Zylinderabschnitts 18 im Bereich des Kolbenschiebers 21 mit Silber beschichtet sein. Auch eine Beschichtung der Feder 23 und/oder 29 mit Silber ist möglich. Gleichfalls kann das untere Endstück des Steigrohrs 13 und/oder ein Teil der Wandung der Durchgangsbohrung 20 mit Silber beschichtet sein. Außerdem ist die der Abgabeöffnung 24 zugewandte Innenseite der Schlußkappe 3 mit Silber beschichtet. Obwohl das Silber im vorliegenden Ausführungsbeispiel als metallisches Silber vorliegt, kann auch ein Silbersalz, wie beispielsweise ein Silberhalogenid, oder ein anderes Schwermetall verwendet werden. Die oligodynamische Wirkung von Schwermetallen, insbesondere von Silber, ist bekannt und braucht hier nicht näher erläutert zu werden. Es sei lediglich erwähnt, daß das Silber in ppb-Konzentrationen in Wasser löst. Die dabei abgegebenen Silberionen wirken bakteriostatisch und bakterizid auf die eindringenden Keime und töten sie auf diese Weise ab.

Die erfindungsgemäße Dosierpumpe arbeitet luftausgleichsfrei, d.h. während ihrer Betätigung erfolgt kein Druckausgleich im Vorratsbehälter 9 durch einströmende Luft.

Die Funktion der erfindungsgemäßen Dosierpumpe ist wie folgt: Wenn der Benutzer nach Entfernen der Verschlußkappe 3 auf den Betätigungskopf 2 drückt und diesen dabei in den zweiten Pumpenkörperabschnitt 5 hineinschiebt, erfolgt gleichzeitig eine entsprechende Bewegung des Kolbens 19 gegen die Kraft der Feder 29. Dadurch wird die Kugel 28 stärker gegen den Ventilsitz 27 gedrückt und die während der vorher gehenden Betätigung der Dosierpumpe in den Innenraum 32 und die Durchgangsbohrung 20 eingesaugte Flüssigkeit 10 unter Druck gesetzt. Dieser Druck verschiebt den Kolbenschieber 21 des Auslaßventils 22 gegen die Kraft der Feder 23, so daß der Anschluß zum Auslaßkanal 25 freigegeben und die Flüssigkeit 10 in genau dosierter Menge durch die Abgabeöffnung 24 abgegeben wird. Sobald der Kolben 19 seinen unteren Todpunkt erreicht hat, fällt der Druck im Innenraum 32 und in der Durchgangsbohrung 20 so stark ab, daß das Auslaßventil 22 schließt und das Einlaßventil 26 öffnet, so daß Flüssigkeit 10 aus dem Vorratsbehälter 9 angesaugt wird. Sodann schließt das Einlaßventil 26 wieder. Daraufhin setzt der Benutzer die Verschlußkappe 3 auf den Betätigungskopf 2 auf und schließt auf diese Weise die Abgabeöffnung 24.

Flüssigkeitsreste an der Abgabeöffnung 24, im Auslaßventil 22, und ggfls. in der Durchgangsbohrung 20, sowie die im Innenraum 32 und im Einlaßventil 29 befindliche Flüssigkeitsmenge gelangen mit den entsprechenden Silberdepots in Berührung, so daß die in ihnen enthaltenen Keime durch die abgegebenen Silberionen abgetötet werden. Auch vom unteren Endstück des Steigrohrs 13 werden Silberionen in die im Vorratsbehälter 9 befindliche Flüssigkeit 10 abgegeben.

## Patentansprüche

1. Fluid-Abgabeeinrichtung für keimfreies Fluid, insbesondere Augentropfen, mit einem Durchgangskanal, der eine Einlaßöffnung für das in einem Vorratsbehälter befindliche Fluid und eine Abgabeöffnung für dasselbe verbindet und in dem eine im Fluid lösbare Substanz mit oligodynamischer Wirkung angeordnet ist, dadurch **gekennzeichnet,**
daß die Fluid-Abgabeeinrichtung als luftausgleichsfrei arbeitende Dosierpumpe mit einem die Einlaßöffnung (15) verschließbaren Einlaßventil (26) ausgebildet und die im Fluid (10) lösbare oligodynamisch wirksame Substanz im Einlaßventil (26) und/oder in dessen Zulauf und/oder Ablauf angeordnet ist.

2. Fluid-Abgabeeinrichtung nach Anspruch 1,
dadurch **gekennzeichnet,**
daß der Durchgangskanal (32,20) zumindest im Bereich des Einlaßventils (26) mit dem Fluid (10) ständig gefüllt ist.

3. Fluid-Abgabeeinrichtung nach Anspruch 1 oder 2,
dadurch **gekennzeichnet,**
daß oligodynamisch wirkende Substanz in einem die Abgabeöffnung (24) verschließbaren Auslaßventil (22) und/oder in dessen Zulauf und/oder Ablauf angeordnet ist.

4. Fluid-Abgabeeinrichtung nach wenigstens einem der vorhergehenden Ansprüche,
dadurch **gekennzeichnet,**
daß oligodynamisch wirkende Substanz an der Innenseite einer auf die Fluid-Abgabeeinrichtung zum Abdecken der Abgabeöffnung (24) aufsetzbare Verschlußkappe (3) angeordnet ist.

5. Fluid-Abgabeeinrichtung nach wenigstens einem der vorhergehenden Ansprüche,
dadurch **gekennzeichnet,**
daß oligodynamisch wirkende Substanz am Verschlußglied (28 bzw. 21) des Einlaßventils (26) und/oder Auslaßventils (22) angeordnet oder zumindest Teil desselben ist.

6. Fluid-Abgabeeinrichtung nach wenigstens einem der vorhergehenden Ansprüche,
dadurch **gekennzeichnet,**
daß oligodynamisch wirkende Substanz an dem mit dem Verschlußglied (28 bzw. 21) des Einlaßventils (26) und/oder Auslaßventils (22) zusammenwirkenden Ventilsitz (27) - bzw. Ventilgehäuse (18 bei 21) angeordnet oder zumindest Teil desselben ist.

7. Fluid-Abgabeeinrichtung nach wenigstens einem der vorhergehenden Ansprüche,
dadurch **gekennzeichnet,**
daß oligodynamisch wirkendsame Substanz an einer das Verschlußglied (28 bzw. 21) des Einlaßventils (26) und/oder Auslaßventils (22) beaufschlagenden Feder (29 bzw. 23) angeordnet oder zumindest Teil desselben ist.

8. Fluid-Abgabeeinrichtung nach wenigstens einem der vorhergehenden Ansprüche,
dadurch **gekennzeichnet,**
daß oligodynamisch wirkende Substanz auf zumindest einem Teilstück des als Steigrohr (13) ausgebildeten Zulaufs des Einlaßventils (26) angeordnet oder zumindest Teil desselben ist.

9. Fluid-Abgabeeinrichtung nach wenigstens einem der vorhergehenden Ansprüche,
dadurch **gekennzeichnet,**
daß oligodynamisch wirksame Substanz im Bereich zwischen beiden Ventilen (22,26) auf zumindest einem Teilstück des Durchgangskanals (32,20) angeordnet oder zumindest Teil desselben ist.

10. Fluid-Abgabeeinrichtung nach wenigstens einem der vorhergehenden Ansprüche,
dadurch **gekennzeichnet,**
daß die oligodynamisch wirksame Substanz in einem Trägermaterial eingelagert ist.

11. Fluid-Abgabeeinrichtung nach Anspruch 10,
dadurch **gekennzeichnet,**
daß das Trägermaterial Korund ist.

12. Fluid-Abgabeeinrichtung nach wenigstens einem der vorhergehenden Ansprüche,
dadurch **gekennzeichnet,**
daß die oligodynamisch wirksame Substanz ein Schwermetall und/oder eine Schwermetall-Legierung ist.

13. Fluid-Abgabeeinrichtung nach Anspruch 12,
dadurch **gekennzeichnet,**
daß das Schwermetall und/oder die Schwermetall-Legierung in metallischer Form verwendet ist.

14. Fluid-Abgabeeinrichtung nach Anspruch 12,
dadurch **gekennzeichnet,**
daß das Schwermetall und/oder die Schwermetall-Legierung in Form einer Verbindung, z.B. als Salz, verwendet ist.

15. Fluid-Abgabeeinrichtung nach wenigstens einem der Ansprüche 12 bis 14,
dadurch **gekennzeichnet,**
daß das Schwermetall Silber ist.

## Claims

1. Fluid delivery device for aseptic fluid, in particular eye drops, having a passage duct which connects an inlet opening for the fluid located in a storage container and a delivery opening for the fluid and in which there is arranged a substance soluble in the fluid and having an oligodynamic effect,
characterized in that
the fluid delivery device is constructed as a dosing pump, operating in a manner which is free of air compensation, with an inlet valve (26) closing the inlet opening (15) and the substance which is soluble in the fluid (10) and which is oligodynamically active is arranged in the inlet valve (26) and/or in its inflow and/or outflow.

2. Fluid delivery device according to claim 1, characterized in that
the passage duct (32, 20) is constantly filled with the fluid (10) at least in the region of the inlet valve (26).

3. Fluid delivery device according to claim 1 or 2, characterized in that
oligodynamically acting substance is arranged in an outlet valve (22) closing the delivery opening (24) and/or in its inflow and/or outflow.

4. Fluid delivery device according to at least one of the preceding claims,
characterized in that
oligodynamically acting substance is arranged at the inner side of a sealing cap (3) which can be attached to the fluid delivery device to cover the delivery opening (24).

5. Fluid delivery device according to at least one of the preceding claims,
characterized in that
oligodynamically acting substance is arranged on the sealing member (28 or 21) of the inlet valve (26) and/or outlet valve (22) or is at least a part of the same.

6. Fluid delivery device according to at least one of the preceding claims,
characterized in that
oligodynamically acting substance is arranged at the valve seat (27) or valve housing (18 at 21) cooperating with the sealing member (28 or 21) of the inlet valve (26) and/or outlet valve (22) or is at least a part of the same.

7. Fluid delivery device according to at least one of the preceding claims,
characterized in that
oligodynamically acting substance is arranged at a spring (29 or 23) loading the sealing member (28 or 21) of the inlet valve (26) and/or outlet valve (22) or is at least a part of the same.

8. Fluid delivery device according to at least one of the preceding claims,
characterized in that
oligodynamically acting substance is arranged on at least one section of the inflow, constructed as a riser (13), of the inlet valve (26) or is at least a part of the same.

9. Fluid delivery device according to at least one of the preceding claims,
characterized in that
oligodynamically active substance is arranged in the region between the two valves (22, 26) on at least one section of the passage duct (32, 20) or is at least a part of the same.

10. Fluid delivery device according to at least one of the preceding claims,
characterized in that
the oligodynamically active substance is incorporated in a carrier material.

11. Fluid delivery device according to claim 10,
characterized in that
the carrier material is corundum.

12. Fluid delivery device according to at least one of the preceding claims,
characterized in that the oligodynamically active substance is a heavy metal and/or a heavy-metal alloy.

13. Fluid delivery device according to claim 12,
characterized in that
the heavy metal and/or the heavy-metal alloy is used in a metallic form.

14. Fluid delivery device according to claim 12,
characterized in that
the heavy metal and/or the heavy-metal alloy is used in the form of a compound, for example as a salt.

15. Fluid delivery device according to at least one of claims 12 to 14,
characterized in that
the heavy metal is silver.

## Revendications

1. Dispositif distributeur de fluide aseptique, en particulier de gouttes pour les yeux, avec un canal de passage reliant une ouverture d'admission pour un fluide se trouvant dans un réservoir et une ouverture d'échappement pour ce fluide, et dans lequel est disposée une substance soluble dans le fluide et ayant une action oligodynamique, **caractérisé** en ce que le dispositif distributeur de fluide est sous la forme d'une pompe doseuse fonctionnant sans rentrées d'air, avec une soupape d'admission (26) fermant l'ouverture d'admission (15), et la substance à action oligodynamique soluble dans le fluide (10) est disposée dans la soupape d'admission (26) et/ou dans le passage d'entrée et/ou de sortie de celle-ci.

2. Dispositif distributeur de fluide selon la revendication 1, caractérisé en ce que le canal de passage (32,20) est constamment rempli de fluide (10), tout au moins dans la zone de la soupape d'admission (26).

3. Dispositif distributeur de fluide selon la revendication 1 ou 2, caractérisé en ce que la substance à action oligodynamique est disposée dans une soupape d'échappement (22) obturant l'ouverture de distribution (24) et/ou dans l'entrée et/ou dans la sortie de cette soupape.

4. Dispositif distributeur de fluide selon au moins une des revendications précédentes, caractérisé en ce que la substance à action oligodynamique est disposée sur la face interne d'un capuchon (3) se posant sur le dispositif distributeur de fluide pour couvrir l'ouverture de distribution (24).

5. Dispositif distributeur de fluide selon au moins une des revendications précédentes, caractérisé en ce que la substance à action oligodynamique est disposée sur l'organe obturateur (28 ou 21) de la soupape d'admission (26) et/ou de la soupape d'échappement (22) ou fait tout au moins partie de cet organe.

6. Dispositif distributeur de fluide selon au moins une des revendications précédentes, caractérisé en ce que la substance à action oligodynamique est disposée sur le siège de soupape (27) coopérant avec l'organe obturateur (28 ou 21) de la soupape d'admission (26) et/ou de la soupape d'échappement (22), ou sur le boîtier de soupape (18 ou 21) ou fait tout au moins partie de ceux-ci.

7. Dispositif distributeur de fluide selon au moins une des revendications précédentes, caractérisé en ce que la substance à action oligodynamique est disposée sur un ressort (29 ou 23) agissant sur l'organe obturateur (28 ou 21) de la soupape d'admission (26) et/ou de la soupape d'échappement (22) ou fait tout au moins partie de ceux-ci.

8. Dispositif distributeur de fluide selon au moins une des revendications précédentes, caractérisé en ce que la substance à action oligodynamique est disposée sur au moins une pièce faisant partie du conduit d'entrée de la soupape d'admission (26) réalisé sous la forme d'un tube plongeur (13), ou fait tout au moins partie de celui-ci.

9. Dispositif distributeur de fluide selon au moins une des revendications précédentes, caractérisé en ce que ladite substance à action oligodynamique est disposée dans la zone entre les deux soupapes (22, 26) sur au moins une partie du canal de passage (32, 20) ou fait tout au moins partie de celle-ci.

10. Dispositif distributeur de fluide selon au moins une des revendications précédentes, caractérisé en ce que la substance à action oligodynamique est incorporée à un matériau porteur.

11. Dispositif distributeur de fluide selon la revendication 10, caractérisé en ce que le matériau porteur est le corindon.

12. Dispositif distributeur de fluide selon au moins une des révendications précédentes, caractérisé en ce que la substance à action oligodynamique est un métal lourd ou un alliage de métal lourd.

13. Dispositif distributeur de fluide selon la revendication 12, caractérisé en ce que le métal lourd et/ou l'alliage de métal lourd est utilisé sous la forme métallique.

14. Dispositif distributeur de fluide selon la revendication 12, caractérisé en ce que le métal lourd et/ou l'alliage de métal lourd est utilisé sous la forme d'une combinaison, par exemple sous la forme d'un sel.

15. Dispositif distributeur de fluide selon au moins une des revendications 12 à 14, caractérisé en ce que le métal lourd est l'argent.
